(19) **Europäisches Patentamt**
**European Patent Office**
**Office européen des brevets**

(11) **EP 3 648 599 B1**

(12) **EUROPEAN PATENT SPECIFICATION**

(45) Date of publication and mention
of the grant of the patent:
**28.01.2026 Bulletin 2026/05**

(21) Application number: **18734835.4**

(22) Date of filing: **05.07.2018**

(51) International Patent Classification (IPC):
***A01N 27/00*** (2006.01)  ***A01P 7/00*** (2006.01)
***A61K 31/01*** (2006.01)

(52) Cooperative Patent Classification (CPC):
**A01N 27/00; A61K 31/01**

(86) International application number:
**PCT/EP2018/068299**

(87) International publication number:
**WO 2019/008116 (10.01.2019 Gazette 2019/02)**

(54) **COMPOUNDS, COMPOSITIONS THEREOF AND METHODS FOR TREATING ECTOPARASITE INFESTATION**

VERBINDUNGEN ZUR BEHANDLUNG VON EKTOPARASITENBEFALL

COMPOSÉS POUR LE TRAITEMENT D'UNE INFESTATION PAR DES ECTOPARASITES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.07.2017 EP 17382439**

(43) Date of publication of application:
**13.05.2020 Bulletin 2020/20**

(73) Proprietor: **Ferrer Internacional, S.A.
08028 Barcelona (ES)**

(72) Inventors:
• **SILVINA BACCHINI, Gabriela
08029 Barcelona (ES)**
• **PUIG ALGORA, Gemma
08950 Esplugues de Llobregat (ES)**

(74) Representative: **ZBM Patents - Zea, Barlocci &
Markvardsen
Rambla de Catalunya, 123
08008 Barcelona (ES)**

(56) References cited:
**EP-A1- 0 226 337       WO-A2-2010/093573
US-A1- 2002 160 029**

• **DAVID C. FLINDERS ET AL: "Pediculosis and
Scabies", AMAERICAN FAMILY PHYSICIAN, vol.
69, no. 2, 15 January 2004 (2004-01-15), pages
341 - 348, XP055403673**
• **SEAN CONATY: "How to Remove a tick with
Vaseline < Dogs :: WonderHowTo", 15 November
2010 (2010-11-15), XP055494842, Retrieved from
the Internet <URL:https://dogs.wonderhowto.
com/how-to/remove-tick-with-vaseline-323144/>
[retrieved on 20180724]**
• **ANONYMOUS: "ICSC 1440 - PETROLATUM
(WHITE)", 30 June 2002 (2002-06-30),
XP055494739, Retrieved from the Internet
<URL:http://www.inchem.org/documents/icsc/
icsc/eics1440.htm> [retrieved on 20180724]**
• **TSUKASA KANII ET AL: "Clinical Evaluations of
Squalane in Patients with Xerotic Dermatoses
and the Results of Patch Test of Squalane and its
Moisturizing Effects", SKIN RESEARCH, vol. 33,
no. 2, 1 January 1991 (1991-01-01), pages 155 -
163, XP055403564, DOI: http://doi.org/10.11340/
skinresearch1959.33.155**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

## Description

This application claims the benefit of European Patent Application EP17382439.2 filed on 06/07/2017.

The present invention relates to compounds for killing ectoparasites and/or their ova on a subject, methods and compositions thereof.

## BACKGROUND OF THE INVENTION

Ectoparasites are organisms which inhabit the skin or outgrowths of the skin of another organism (the host) for various periods, and may be detrimental to the latter. Various ectoparasites cause significant infestations in humans and in many kinds of domestic animals including livestock, pets, laboratory animals, poultry, fish and bees. Many of these ectoparasites (e.g. most lice) are host specific, while others (e.g. many ticks) parasitize a wider range of hosts. Many ectoparasites are known to be vectors of pathogens, which the parasites typically transmit to hosts while feeding or (occasionally) defecating. Examples of ectoparasites are lice, ticks, mites and fleas.

Lice are external parasites of warm blooded animals. Humans are host to three different types of lice: headlice, body lice, and crabs or pubic lice.

Head lice infestation (*pediculosis capitis*) is a persistent problem with as many as 6-12 million people worldwide affected each year. The problem is particularly prevalent in children with preschool and elementary-age children aged 3-10 and their families becoming infested most often.

Head lice infestation is produced by the common head louse *Pediculus humanus capitis,* and typically causes itching of the scalp. As feed on human blood, they may cause lesions to develop on the scalp, swollen glands on the neck or under arms, or other symptoms. Head lice infestation causes serious problems due to the negative social implications of the infestation. Body lice (*Pediculus humanus corporis)* are also bothersome to humans and carry the additional hazard of being the vectors of certain diseases, such as exanthematic or epidemic typhus and recurrent fever.

Life cycle of the head louse falls into three phases: egg, nymph and mature louse.

The louse's hard chitinous exoskeleton serves as protection from external elements. Lice eggs (or ova) are similarly protected by a chitinous sheath surrounding the eggs and attached to the hair shaft. In the case of lice these ova are also referred to as nits. Most eggs take 7 days to hatch (but a few may take longer, up to 13 days) and may appear visible for weeks after death of the egg. Although lice may be affected by the use of an insecticide, the eggs often remain resistant to attack. Thus, optimum treatment of a lice infestation includes both a pediculicide, which kills the adult lice and nymphs, and an ovicide, which interrupts the development of the eggs.

Treatment options for head lice infestation can broadly be divided into five groups as follows: topically applied insecticides; topically applied physically acting agents; topically applied homeopathic, plant formulations and other remedies; oral drugs; and mechanical agents (combs, electronic devices, heating devices). Various of these compositions are available for treating lice infestations, which generally take a topical approach to treatment. Most of these treatments involve the use of insecticides that are harsh agents, thus raising toxicity concerns. Lice can also become resistant to the insecticides used and therefore the compositions can lose their effectiveness over time.

Eradication of head lice involves total removal or destruction of the mature lice, nymphs and the eggs on each host. Various attempts have been proposed to achieve such destruction but none are wholly satisfactory.

US 4,147,800 A discloses that aliphatic carbocyclic esters exhibit pediculicidal properties if used at concentrations above 70 % of the composition and teaches the use of these esters in combination with aliphatic alcohols.

US 6,303,581 teaches a variety of surfactants, including non-volatile lipids, non-volatile fatty alcohols and non-volatile fatty esters or mixtures thereof, that are water-soluble or aqueous-based and have been found to be pediculostatic.

GB 2204243B2 discloses a method for controlling lice or their ova on human subjects consisting of applying a topical composition comprising a lousicide and at least a C2-C4 alkyl ester of a C10-C20 fatty acid dissolved in an alcoholic solution. Preferably the lousicide comprises carbaryl, malathion or phenothrin.

WO00/01347 discloses compositions containing spinosad, siloxanes and fatty acid esters.

WO2001019190 discloses compositions that contain 85 to 99.9 % cyclosiloxanes as pediculicides.

WO2001040446 discloses the ectoparasicidal activity of a composition comprising isopropylmyristate, octyl palmitate and ivermectin.

WO03092583 discloses compositions for killing ectoparasites on a subject containing a fatty acid ester, e.g., isopropyl myristate, effective for killing ectoparasites. Also described are compositions containing a fatty acid ester and a siloxane (e.g. decacyclomethicone).

US2013/0018016A1 discloses a composition for killing ectoparasites and/or their eggs, comprising at least one volatile at room temperature, liquid, non-polar organic solvent, 1 to 10 wt.-%, based on the total composition, of at least one spreading agent and 35 to 65 wt.-%, based on the total composition, of at least one polysiloxane having a viscosity of greater than 90 cSt.

David C. Flinders et al., in "Pediculosis and Scabies", American Family Physician, 2004, vol. 69, pp.342-344, discloses the use of olive oil or petroleum ointment against nits.

The need for further treatment increases the exposure to these harsh agents and increases the cost. Additionally, clinicians and parents are reluctant to treat children with agents that can also prove toxic to human beings. There are reports in the medical literature, for example, that children treated with lindane have developed seizures. Moreover, many of these compounds have unpleasant odours or other undesirable properties, causing non-compliance by the patient, leading to re-infestation of the individual, and spreading of the infestation to others. In addition, the harshness of these agents make them unsuitable for use as prophylactics.

Thus, there remains a need in the art for methods and kits useful for treating and removing ectoparasites infestations, such as lice, ticks, mites or fleas infestations, that are easy to use, cosmetically attractive and effective against ectoparasites resistant to other treatments. Moreover, there remains a need for methods and kits that eradicate nits, also referred to as eggs or ova, as well as adults and instars, and that can be used prophylactically to prevent initial infection or re-infestation. Accordingly, these are some of the objects of the present invention. Thus, the present invention has been made in view of the above problems and it is an object of the invention to provide a safe and effective method of eradicating ectoparasites, especially lice, ticks, mites or fleas. In particular, to provide an effective and pediculicidal and/or ovicidal composition for treatment of lice infestations, more specifically head lice infestations. It is a further object of this invention to provide a compound or composition which will be effective in the prevention of ectoparasites infestations, such as lice, ticks mites or fleas infestations, especially head lice infestations.

The above issues have been addressed in the present invention and specific embodiments thereof.

## DESCRIPTION OF THE INVENTION

The present invention provides squalane for use in killing lice and/or their ova on a mammal subject. It also provides compositions for killing non-flying ectoparasites, and/or their ova on a subject, preferably a mammal subject, the subject is a human and the non-flying ectoparasites are lice.

The inventors discovered unexpectedly that the compound squalane is particularly effective in killing lice on a subject as well as their ova. The present inventors also discovered that the combination of squalane and silicones (e.g., a cyclomethicone) is also useful for killing lice. Thus, squalane alone or squalane in combination with silicones may be included in a composition in an amount effective for killing lice to result in a composition that is effective for this purpose.

In a further embodiment of the first aspect, squalane is for use in killing lice and/or their ova on a subject. Squalane is effective in killing ectoparasites as well as their ova, meaning one or the other or both.

Squalane is frequently used in cosmetics as emollient and moisturizer. However, there is no indication in the prior-art that has any effect on ectoparasites. Squalane has low acute toxicity and is not an irritant at the concentrations used in cosmetics (up to 100%). Squalane, a saturated aliphatic hydrocarbon (C30H62), is obtained by hydrogenation of squalene, which has been used in cosmetics as a moisturizer. Squalene, a precursor for squalane, is contained in shark liver oil, rice, olive, soybean, and so on. Squalane (2,6,10,15,19,23-hexamethyltetracosane) is a clear, odourless and colourless oil of very low polarity. It has a density (20 °C) of 0.800 - 0.815 g/cm$^3$.

According to this invention, by the term "ectoparasite", it is meant a parasite that lives outside the body of the host, on the skin or outgrowths of the skin of the host.

In another preferred embodiment, the mammal is a human. In a most preferred embodiment the lice, are head lice.

The term "subject" includes plants and mammals. The term "mammal" includes humans, and also includes animals that are members of the class Mammalia. This will usually be a human, but it also includes pets such as dogs, cats, ferrets, rabbits, gerbils, and guinea pigs. Mammals also include domestic animals such as bovines, porcines, ovines, and equines. While most fur-bearing animals can become infested with fleas and ticks, pigs, horses, and cattle can also be infested with lice (e.g., the Haematopinus suis, which infests pigs, and other Haematopinus spp. that infest horses and cattle). These infestations are treatable with the product and compositions described herein.

By "infestation", it is meant the presence of lice, fleas, ticks, mites or other ectoparasites that are the target of the treatment. Ectoparasites or pests include, but are not limited to, head lice, body lice (e.g., Pediculus humanus), crab lice (e.g., Phthirus pubis), mites (scabies), fleas and ticks. The presence of eggs of the target ectoparasite also constitutes infestation. Thus, optimum treatment of an ectoparasites infestation includes both the eradication of adult parasites and their eggs (ova). In the case of lice infestation, it includes both a lousicidal effect, which kills the adult lice and nymphs, and an ovicidal effect, which interrupts the development of the eggs. In a preferred embodiment, the ectoparasite egg or ova is a louse egg, more preferably head louse egg.

As regards crab lice, humans are the only known host, although a closely related species, *Phthirus gorillae,* is known to infect gorilla populations. The human parasite diverged from *Phthirus gorillae* approximately 3.3 million years ago, and is more distantly related to the genus Pediculus, which contains human and body lice, although they all share the same suborder Anoplura, and thus have important common features.

Despite differences between human lice, they are commonly treated with over-the-counter (OTC) products containing agents such as permethrin, pyrethrins, malathion and products that physically suffocate the ectoparasite. In fact, the guidelines for chosing the pediculicide to be used against body and pubic lice are similar to those used for head lice.

In a second aspect of the first invention, it is provided a composition for use in killing lice and/or their ova on a subject comprising squalane, wherein squalane is present in an amount of at least 5 % by weight in respect of the total amount of the composition.

The term "% by weight" in the present invention refers to % **w/w** (weight per weight) in respect of the total amount of the composition.

The compositions as herein disclosed offer the surprising and highly desirable combination of benefits of being highly effective, spreading evenly, drying quickly, having low mammalian toxicity, and being hair and skin compatible by not having a significantly greasy or oily texture. Therefore, the compositions of the present invention eliminate the disadvantages of previously available compositions of being messy and inconvenient to apply, emitting an unpleasant odour, having limited effectiveness, or having substantial mammalian toxicity.

The compositions do not require the presence of any alcohol since the treated patient will have bites and lesions on the scalp or body caused by the ectoparasites, and the application of compositions containing alcohols will cause pain and discomfort. Thus, the compositions do not need to contain aliphatic alcohols or any other alcohols. The compositions as herein disclosed have shown ovicidal (i.e. they interrupt egg/ova development and kill eggs/ova), lousicidal (i.e. they kill lice), or pediculicidal (i.e. they kill kill both eggs and lice) activity or efficacy.

In a further embodiment of the second aspect, the composition for use does not comprise Lippia javanica essential oil. As used herein, "Lippia javanica oil" refers to the oil from a species of the family Verbenaceae, which is a family of herbs and shrubs or small trees that have aromatic leaves. It includes oil from the species Lippia javanica (Burm f.) Spreng. It is known by its common name, fever tree. Lippia javanica oil is also known as zinziba oil. Lippia javanica grows in South Africa, Swaziland, Zambia, Botswana, Kenya, Malawi, Tanzania and Mozambique.

In a preferred embodiment of the composition as herein disclosed, the total amount of squalane present in the composition ranges from 5 to 20 % by weight in respect of the total amount of the composition. Preferably, squalane is present in an amount of more than 20 % by weight in respect of the total amount of the composition.

In a preferred embodiment of the composition as herein disclosed, squalane is present in an amount of at least 30% by weight in respect of the total amount of the composition, preferably in an amount of at least 40 % by weight in respect of the total amount of the composition, more preferably in an amount of at least 50 % by weight in respect of the total amount of the composition, even more preferably in an amount of at least 60 % by weight in respect of the total amount of the composition.

In another preferred embodiment, the mammal is a human. In a most preferred embodiment, the ectoparasites are head lice.

In a further embodiment of the composition as herein disclosed, said composition further comprises at least a siloxane. Said siloxane is preferably a cyclic siloxane, preferably the cyclic siloxane is selected from cyclomethicone, hexamethylcyclotrisiloxane, octamethylcyclotetrasiloxane, dodecamethylcyclohexasiloxane, decamethylcyclopentasiloxane, hexamethyldisiloxane and mixtures thereof, more preferably the composition comprises decamethylcyclopentasiloxane.

Siloxanes are useful in the present invention. A siloxane is a volatile, cyclic, non-polymeric silicone that dries quickly, spreads evenly, and does not leave a greasy residue. Cyclomethicones are a group of methyl siloxanes, a class of liquid silicones (cyclic polydimethylsiloxane polymers) that possess the characteristics of low viscosity and high volatility as well as being skin emollients and in certain circumstances useful cleaning solvents.

The USP32-NF27 describes cyclomethicone as a fully methylated cyclic siloxane containing repeating units of the formula $[-(CH_3)_2SiO-]_n$ in which n is 4, 5, or 6, or a mixture of them. The cyclomethicone used in the present invention is clear, colourless, volatile carrier that provides a soft silky feel. It is composed preferably mainly of decamethylcyclopentasiloxane (D5), which is present at greater than 97%. The octamethylcyclotetrasiloxane (D4) is present at values less than 0.9% in cyclomethicone.

In a preferred embodiment of the composition as herein disclosed, the total amount of siloxane or siloxanes present in the composition ranges from 30 to 70 % by weight in respect of the total amount of the composition, preferably ranges from 35 to 65 % by weight in respect of the total amount of the composition, more preferably ranges from 40 to 55 % by weight in respect of the total amount of the composition.

In a further embodiment of the composition as herein disclosed, said composition comprises at least a further emollient. Said further emollient is preferably a fatty acid ester, more preferably selected from cetyl palmitate, decyl oleate, ethyl oleate, isopropyl myristate, isopropyl palmitate, ethylhexyl stearate and mixtures thereof, even more preferably the composition comprises ethylhexyl stearate (i.e. 2-ethylhexyl stearate). Ethylhexyl stearate is colourless to slightly yellow liquid ester with medium spreading properties. It has a density approx. of 0.85 $g/cm^3$.

By "fatty acid ester", it is meant a type of ester that results from the combination of a fatty acid with an alcohol, e.g. isopropyl myristate. "Fatty acid" refers to a carboxylic acid with an aliphatic chain containing a number of carbon atoms equal to or higher than 4, which is either saturated or unsaturated. Most naturally occurring fatty acids have an unbranched chain of an even number of carbon atoms, from 4 to 28. An ester is a functional derivative of a carboxylic acid, where the -OH group of the carboxylic acid has been replaced by an -OR, R being an alkyl group.

In a preferred embodiment of the composition as herein disclosed, the total amount of fatty acid ester or fatty acid esters present in the composition ranges from 1 to 10 % by weight in respect of the total amount of the composition, preferably ranges from 3 to 7 % by weight in respect of the total amount of the composition.

In a preferred embodiment of the composition as herein disclosed, the total amount of squalane present in the composition is present in an amount of more than 20 % by weight in respect of the total amount of the composition, the ectoparasites are head lice, the composition comprises decamethylcyclopentasiloxane in an amount that ranges from 35 to 65 % by weight in respect of the total amount of the composition and the composition comprises ethylhexyl stearate in an amount that ranges from 3 to 7 % by weight in respect of the total amount of the composition.

In a further embodiment of the composition as herein disclosed, said composition further comprises tea tree oil. In a preferred embodiment, the total amount of tea tree oil present in the composition ranges from 0.5 to 5 % by weight in respect of the total amount of the composition, preferably ranges from 1 to 3 % by weight in respect of the total amount of the composition. As used herein, tea tree oil refers to Tea Tree (Melaleuca alternifolia) oil. "Melaleuca alternifolia oil" refers to the essential oil from the bottlebrush tree and is commonly called tea tree oil. Melaleuca alternifolia is indigenous to New Zealand and Australia but also grows in Southern California. The oil usually is produced by distillation of the leaves. Melaleuca alternifolia oil has a medicinal odour although some people characterize the smell as a pungent spicy woodsy scent. The chemical constituents of Melaleuca alternifolia oil include various levels of [alpha]-pinene, sabinene, [alpha]-terpinene, limonene, p-cymene, 1,8-cineole, [gamma]-terpinene, terpinolene, terpinen-4-ol, [alpha]- terpineol, aromadendrene, d-cadinene, globulol, and viridiflorol. The oil is commercially available (e.g., see J. Rose, The Aromatherapy Book - Applications & Inhalations (North Atlantic Books, 1992); and from Berje Essential Oils, Bloomfield, NJ; Liberty Natural Products, Portland, OR; and Mountain Rose Herbs, Eugene, OR).

In a further embodiment, the composition as herein disclosed comprises at least one further ingredient which is selected from the group consisting of Ribes nigrum (blackcurrant) seed oil, Helianthus annuus seed oil, Helianthus annuus seed oil unsaponifiables, Cardiospermum halicacabum flower, leaf or vine extract, Rosmarinus officinalis (rosemary) plant, flower or leaf extract, Magnolia officinalis bark extract, Magnolia grandiflora bark extract, Vitis vinifera seed extract (e.g. caprylic/capric triglyceride), Echium plantagineum seed oil, Centella asiatica extract, Camelia sinensis leaf extract, Melia azadirachta leaf extract, Withania somnifera root extract, Aloe barbadensis extract (preferably leaf juice), corn (zea mays) oil, linoleic acid, linolenic acid, oleic acid, palmitic acid, stearic acid, eicosapentaenoic acid (i.e. EPA), docosahexaenoic acid (i.e. DHA), azelaic acid, myristic acid and any mixture thereof. Preferably, said composition comprises at least one further ingredient which is selected from the group consisting of Ribes nigrum (blackcurrant) seed oil, Helianthus annuus seed oil, Helianthus annuus seed oil unsaponifiables, Cardiospermum halicacabum flower, leaf or vine extract, Rosmarinus officinalis (rosemary) plant, flower or leaf extract, Magnolia officinalis bark extract, Vitis vinifera seed extract (e.g. caprylic/capric triglyceride), Echium plantagineum seed oil, corn (zea mays) oil, linoleic acid, linolenic acid and any mixture thereof. Preferably, the total amount of said at least one further ingredient present in the composition ranges from 0.2 to 5% by weight in respect of the total amount of the composition, more preferably the total amount of said at least one further ingredient present in the composition ranges from 0.5 to 4% by weight in respect of the total amount of the composition.

Said at least one further ingredient more preferably comprises Ribes nigrum (blackcurrant) seed oil, Helianthus annuus seed oil and Rosmarinus officinalis (rosemary) plant, flower or leaf extract; more preferably, said at least one further ingredient comprises Ribes nigrum (blackcurrant) seed oil, Helianthus annuus seed oil, Rosmarinus officinalis (rosemary) plant, flower or leaf extract and Cardiospermum halicacabum flower, leaf or vine extract; even more preferably, said at least one further ingredient comprises Ribes nigrum (blackcurrant) seed oil, Helianthus annuus seed oil, Helianthus annuus seed oil unsaponifiables, Rosmarinus officinalis (rosemary) plant, flower or leaf extract and Cardiospermum halicacabum flower, leaf or vine extract.

In another preferred embodiment, said at least one further ingredient comprises Magnolia officinalis bark extract and Vitis vinifera seed extract (e.g. caprylic/capric triglyceride). Also in a preferred embodiment, said at least one further ingredient may comprise Helianthus annuus seed oil, Cardiospermum halicacabum flower, leaf or vine extract, and Echium plantagineum seed oil.

Alternatively, said at least one further ingredient may preferably comprise linoleic acid (i.e. omega-6 essential fatty acid) and linolenic acid (i.e. omega-3 essential fatty acid).

The presence of any of these herbal or botanical extracts and/or essential fatty acids in this preferred embodiment may advantageously contribute to improve skin condition, particularly scalp condition, in subjects with skin hypersensitivity, dry skin or atopic skin, who tend to suffer from itching, a burning feeling, reddening of the skin or even damaged skin surface. Without wishing to be bound by theory, it is believed that such at least one further ingredient may contribute to support skin condition during the therapeutic treatment provided provided by the compositions of the present invention, that is, during the killing of ectoparasites and/or their ova on a subject, particularly lice.

In a further embodiment of the composition as herein disclosed, the composition consists only of squalane.

The compositions of the invention are intended to expose the ectoparasites for a period of time to them. After a given time of this exposure ectoparasites and/or their ova are killed. The compositions of the invention are formulated to be applied on the zone infested by ectoparasites.

Preferably, the compositions are formulated to be applied to the scalp of a subject suffering from a head lice infestation and are left on the treated subject for a period of time. In a further embodiment of the composition as herein disclosed, at least 75% of ectoparasites present are killed within 4 hours after a 10-minute exposure to the composition, preferably at least 75% of ectoparasites present are killed within 2 hours after a 10-minute exposure to the composition, preferably at least 75% of ectoparasites present are killed within 1 hour after a 10-minute exposure to the composition, more preferably at least 75% of ectoparasites present are killed within 30 minutes after a 10-minute exposure to the composition, even more preferably at least 75% of ectoparasites present are killed within 5 minutes after a 5-minute exposure to the composition.

By the term "killed", it is meant that ectoparasites, for instance lice, do not show any major vital signs. The criterion for the ectoparasiticidal efficacy (pediculicidal in the case of lice) of the test preparations was the mortality rate within each group of parasites - assessed by strictly defined criteria for vitality. Only when there was a total absence of vital signs or when there were only minimal vital signs such as gut movements or movements of the antennae ("minor vital signs") the parasites were declared to be dead. In the same manner, ovicidal efficacy was evaluated when there is no hatch at this stage.

In a preferred embodiment of the composition as herein disclosed, at least 80% of ectoparasites present are killed within 5 min after a 5-minute exposure to the composition, preferably at least 90% of ectoparasites present are killed within 5 minutes after a 5-minute exposure to the composition, more preferably at least 100% of ectoparasites present are killed within 5 minutes after a 5-minute exposure to the composition.

In a more preferred embodiment of the composition as herein disclosed, at least 80% of ectoparasites present are killed within 5 min after a 2-minute exposure to the composition, preferably at least 90% of ectoparasites present are killed within 5 minutes after a 2-minute exposure to the composition, more preferably at least 100% of ectoparasites present are killed within 5 minutes after a 2-minute exposure to the composition.

The ectoparasites are adult lice, more preferably adult head lice.

The compositions as herein disclosed are also highly effective in killing the lice ova. In a further embodiment of the composition as herein disclosed, at least 60% of the ova are killed within 14 days after 10-minute exposure, preferably at least 80% of the ova are killed within 14 days after 10-minute exposure, more preferably at least 80% of the ova are killed within 14 days after 5-minute exposure, even more preferably at least 40% of the ova are killed within 14 days after 2-minute exposure.

In a further embodiment of the second aspect of the first invention, a composition which does not comprise any siloxane, but comprises at least one carrier, is provided. This siloxane-free composition is suitable for use in killing ectoparasites and/or their ova on a subject while, at the same time, contributes to reducing environmental hazards typically associated to the persistent nature of siloxanes, which have also increasingly been in focus because of their bioaccumulation, especially in marine environments.

Preferred examples of said at least one carrier include, but are not limited to, isododecane, isohexadecane, hydrogentated polydecene, C13-C15 alkane, isodecyl neopentanoate, neopentylglycol heptanoate, glycol distearate, dicaprylyl carbonate, diethylhexyl carbonate, propylene glycol n-butyl ether, ethyl-3-ethoxypropionate, propylene glycol methyl ether acetate, tridecyl neopentanoate, propylene glycol methylether acetate (PGMEA), propylene glycol methylether (PGME), octyldodecyl neopentanoate, diisobutyl adipate, diisopropyl adipate, propylene glycol dicaprylate/dicaprate, octyl palmitate, hydrogenated castor oil behenyl ester, hydrogenated castor oil cetyl ester, hydrogenated castor oil stearyl ester, hydrogenated ethylhexyl olivate, hydrogenated olive oil unsaponifiables, hydrogenated ethylhexyl sesamate, hydrogenated isocetyl olivate, hydrogenated isopropyl jojobate, cetearyl olivate/sorbitan olivate, decyl olivate, ethylhexyl olivate, octyldodecyl olivate, stearyl olivate and any mixture thereof.

More preferably, the total amount of said at least one carrier ranges from 50 to 75% by weight in respect of the total amount of the composition, preferably ranges from 55 to 70% by weight in respect of the total amount of the composition, and more preferably ranges from 60 to 65% by weight in respect of the total amount of the composition.

In a preferred embodiment, the composition comprises isohexadecane and isododecane, more preferably comprises isohexadecane, isododecane and a C13-C15 alkane, and still more preferably comprises isohexadecane, isododecane, a C13-C15 alkane and diethylhexyl carbonate.

In another preferred embodiment, the composition comprises diethylhexyl carbonate, hydrogenated ethylhexyl olivate and optionally also hydrogenated olive oil unsaponifiables.

In a third aspect of the first invention, it is provided a topical composition comprising squalane and at least a siloxane.

In a further embodiment of the topical composition of the third aspect, squalane is present in an amount of at least 30% by weight in respect of the total amount of the composition, preferably in an amount of at least 40 % by weight in respect of the total amount of the composition, more preferably in an amount of at least 50 % by weight in respect of the total amount of the composition, even more preferably in an amount of at least 60 % by weight in respect of the total amount of the composition.

In a further embodiment of the topical composition as herein disclosed, the siloxane is a cyclic siloxane, preferably the cyclic siloxane is selected from cyclomethicone, hexamethylcyclotrisiloxane, octamethylcyclotetrasiloxane, dodecamethylcyclohexasiloxane, decamethylcyclopentasiloxane, hexamethyldisiloxane and mixtures thereof, more preferably the composition comprises decamethylcyclopentasiloxane. In a preferred embodiment, the total amount of siloxane or siloxanes present in the composition ranges from 30 to 70 % by weight in respect of the total amount of the composition, preferably ranges from 35 to 65 % by weight in respect of the total amount of the composition, more preferably ranges from 40 to 55 % by weight in respect of the total amount of the composition.

In a further embodiment of the topical composition as herein disclosed, the composition comprises at least a further emollient. Said further emollient is preferably a fatty acid ester, more preferably selected from cetyl palmitate, decyl oleate, ethyl oleate, isopropyl myristate, isopropyl palmitate, ethylhexyl stearate and mixtures thereof, even more preferably the composition comprises ethylhexyl stearate. In a preferred embodiment, the total amount of fatty acid ester or fatty acid esters present in the composition ranges from 1 to 10 % by weight in respect of the total amount of the composition, preferably ranges from 3 to 7 % by weight in respect of the total amount of the composition.

In a further embodiment of the topical composition as herein disclosed, said composition further comprises tea tree oil. In a preferred embodiment, the total amount of tea tree oil present in the composition ranges from 0.5 to 5% % by weight in respect of the total amount of the composition, preferably ranges from 1 to 3 % by weight in respect of the total amount of the composition.

In a further embodiment, the topical composition as herein disclosed comprises at least one further ingredient which is selected from the group consisting of Ribes nigrum (blackcurrant) seed oil, Helianthus annuus seed oil, Helianthus annuus seed oil unsaponifiables, Cardiospermum halicacabum flower, leaf or vine extract, Rosmarinus officinalis (rosemary) plant, flower or leaf extract, Magnolia officinalis bark extract, Magnolia grandiflora bark extract, Vitis vinifera seed extract (e.g. caprylic/capric triglyceride), Echium plantagineum seed oil, Centella asiatica extract, Camelia sinensis leaf extract, Melia azadirachta leaf extract, Withania somnifera root extract, Aloe barbadensis extract (preferably leaf juice), corn (zea mays) oil, linoleic acid, linolenic acid, oleic acid, palmitic acid, stearic acid, eicosapentaenoic acid (i.e. EPA), docosahexaenoic acid (i.e. DHA), azelaic acid, myristic acid and any mixture thereof. Preferably, said composition comprises at least one further ingredient which is selected from the group consisting of Ribes nigrum (blackcurrant) seed oil, Helianthus annuus seed oil, Helianthus annuus seed oil unsaponifiables, Cardiospermum halicacabum flower, leaf or vine extract, Rosmarinus officinalis (rosemary) plant, flower or leaf extract, Magnolia officinalis bark extract, Vitis vinifera seed extract (e.g. caprylic/capric triglyceride), Echium plantagineum seed oil, corn (zea mays) oil, linoleic acid, linolenic acid and any mixture thereof.

Preferably, the total amount of said at least one further ingredient present in the composition ranges from 0.2 to 5% by weight in respect of the total amount of the composition, more preferably the total amount of said at least one further ingredient present in the composition ranges from 0.5 to 4% by weight in respect of the total amount of the composition.

Said at least one further ingredient more preferably comprises Ribes nigrum (blackcurrant) seed oil, Helianthus annuus seed oil and Rosmarinus officinalis (rosemary) plant, flower or leaf extract; more preferably, said at least one further ingredient comprises Ribes nigrum (blackcurrant) seed oil, Helianthus annuus seed oil, Rosmarinus officinalis (rosemary) plant, flower or leaf extract and Cardiospermum halicacabum flower, leaf or vine extract; even more preferably, said at least one further ingredient comprises Ribes nigrum (blackcurrant) seed oil, Helianthus annuus seed oil, Helianthus annuus seed oil unsaponifiables, Rosmarinus officinalis (rosemary) plant, flower or leaf extract and Cardiospermum halicacabum flower, leaf or vine extract.

In another preferred embodiment, said at least one further ingredient comprises Magnolia officinalis bark extract and Vitis vinifera seed extract (e.g. caprylic/capric triglyceride). Also in a preferred embodiment, said at least one further ingredient may comprise Helianthus annuus seed oil, Cardiospermum halicacabum flower, leaf or vine extract, and Echium plantagineum seed oil.

Alternatively, said at least one further ingredient may preferably comprise linoleic acid (i.e. omega-6 essential fatty acid) and linolenic acid (i.e. omega-3 essential fatty acid).

The presence of any of these herbal or botanical extracts and/or essential fatty acids in this preferred embodiment may advantageously contribute to improve skin condition, particularly scalp condition, in subjects with skin hypersensitivity, dry skin or atopic skin, who tend to suffer from itching, a burning feeling, reddening of the skin or even damaged skin surface. Without wishing to be bound by theory, it is believed that such at least one further ingredient may contribute to support skin condition during the therapeutic treatment provided provided by the compositions of the present invention, that is, during the killing of ectoparasites and/or their ova on a subject, particularly lice.

More preferred formulations of the present invention are comprised of a 50:45:5 mixture of a squalane, cyclomethicone and ethylhexyl stearate (w/w).

In a further embodiment of the topical composition as herein disclosed, the composition is for use in killing ectoparasites and/or their ova on a subject. In a preferred embodiment, squalane is the sole ectoparasiticidal agent present in the composition.

In a fourth aspect of the first invention, it is provided a method of killing ectoparasites, preferably non-flying ectoparasites and/or their ova on a subject comprising, topically administering squalane to an area on the subject where ectoparasites or their ova are present.

In a fifth aspect of the first invention, it is provided a method of killing ectoparasites, preferably non-flying ectoparasites and/or their ova on a subject comprising, topically administering to an area on the subject where ectoparasites or their ova are present the composition of the second aspect of the first invention.

The methods disclosed herein include topically administering a composition of the invention to an area on the human where ectoparasites are present. As noted above, the compositions preferably remain in contact with the treated area for a period of time.

By the term "topical application", it is meant that the composition is applied to the exterior of the treated subject, e.g. to the exterior skin, hair, fur, or foliage. This application includes, but is not limited to, manual application or application by various automated means, for example, spraying or painting onto a treated subject, or other means. In some embodiments, an ectoparasiticidal composition, for instance a pediculicidal composition, according to the present invention may be applied to the hair or skin of a subject in need thereof. The ectoparasiticidal composition, for instance a pediculicidal composition, may be applied topically in the form of a solution, cream, emulsion, lotion, gel, spray, ointment or foam.

In a further embodiment of the method of the fourth and fifth aspects, the composition is applied for 30 minutes or less, preferably the composition is applied for 20 minutes or less, more preferably the composition is applied for 10 minutes or less, even more preferably the composition is applied for 5 min or less. In a preferred embodiment, the composition is applied for 2 minutes.

In a further embodiment of the fourth and fifth aspects, the ectoparasites are non-flying ectoparasites. Said ectoparasites are preferably selected from the group consisting of lice, mites, ticks and fleas. In a preferred embodiment, the subject is a mammal. Said mammal is preferably a human. In a more preferred embodiment, the ectoparasites are lice, preferably head lice.

In another aspect of the first invention, it is provided a kit for use in treating lice infestations comprising squalane or the composition of the second or third aspect of the first invention in a package or other enclosure; and instructions describing how to use the items included in the kit to kill lice.

In various embodiments, the kits include a composition of the present invention in a package or other enclosure. In a further embodiment of the kit of the eighth aspect, the kit further comprises a nit comb to assist in removing lice and their eggs from hair. The "nit comb" is an ordinary comb for ordering hair by passing it through the hair. For example, the OTC ANTIPIOJOS® nit comb (Ferrer Internacional SA, Spain), MEDI-SWEEP Lice Comb (Classic Products, Oxnard, CA) are preferred embodiments of the lice comb to be included in the kit. The package can be a box, or may simply be a wrapping (preferably of carton) that surrounds the kit. The comb is preferably provided inside the package, but can also be attached to the outside of the package. In other embodiments, the kits include shower cap. In preferred embodiments, the kit also contains instructions that describe how to use the items included in the kit to kill ectoparasites.

The inventors discovered unexpectedly that aliphatic hydrocarbons are particularly effective in killing ectoparasites on a subject as well as their ova. The present inventors also discovered that the combination said aliphatic hydrocarbons and silicones (e.g., a cyclomethicone) also can kill ectoparasites. Said compounds may be included in a composition in an amount effective for killing lice, fleas, ticks, mites and other ectoparasites to result in a composition that is effective for this purpose.

In a second aspect of the second invention, it is provided a composition for use in killing lice and/or their ova on a mammal subject comprising an aliphatic hydrocarbon compound having a density of 0.7 to 0.9 $g/cm^3$ at 20 °C and at least a siloxane having a viscosity of less than 90 cSt.

The present invention provides compositions that are useful for treating ectoparasites on a mammal. The inventors discovered unexpectedly that just an aliphatic hydrocarbon compound having a density of 0.7 to 0.9 $g/cm^3$ at 20 °C and at least a siloxane having a viscosity less than 90 cSt have the effect of killing lice and/or their ova.

The term "density" of a substance refers to the mass of a unit volume of a substance at 20°C. Density is usually listed in grams per cubic centimeter.

The viscosity of a fluid is a measure of its resistance to gradual deformation by shear stress or tensile stress. For liquids, it corresponds to the informal concept of "thickness". The term "viscosity" refers to the value measured at 25°C. The term viscosity in the present invention it refers to kinematic viscosity. The term "kinematic viscosity" refers to a viscosity which is determined by measuring the dynamic viscosity $\mu$, and dividing the dynamic viscosity $\mu$ by the density of the fluid $\rho$, of the liquid measured at the same temperature. The unit of kinematic viscosity can be expressed by centistokes (cSt) equalling 1 mm2 /sec. Kinematic viscosity can be determined using the methods according to European. Pharmacopeia (Ph. Eur.) online 9.3, chapter (2.2.9) Capillary viscometer method and (2.2.49) Falling ball and automatic rolling ball viscometer methods.

The hydrocarbon compound is present in an amount of at least 5 % by weight in respect of the total amount of the composition. In a preferred embodiment, the hydrocarbon compound is present in an amount of at least 20% by weight in respect of the total amount of the composition, preferably in an amount of at least 30% by weight in respect of the total amount of the composition, preferably in an amount of at least 40 % by weight in respect of the total amount of the composition, more preferably in an amount of at least 50 % by weight in respect of the total amount of the composition and even more preferably, even more preferably in an amount of at least 60 % by weight in respect of the total amount of the composition.

The subject is a mammal, preferably the mammal is a human. In another preferred embodiment, the ectoparasites are head lice.

The hydrocarbon is preferably a fluid to semi-solid alkane. Said hydrocarbon compound is preferably selected from liquid paraffin, solid paraffin, petroleum jelly, squalane, squalene, isohexadecane, isoeicosane, isododecane and mixtures thereof. Preferably, the hydrocarbon is squalane.

In a further embodiment of the composition of the second invention, the low-viscosity siloxane is a cyclic siloxane, preferably the cyclic siloxane is selected from cyclomethicone, hexamethylcyclotrisiloxane, octamethylcyclotetrasiloxane, dodecamethylcyclohexasiloxane, decamethylcyclopentasiloxane, hexamethyldisiloxane and mixtures thereof, more preferably the composition comprises decamethylcyclopentasiloxane. In a preferred embodiment, the total amount of siloxane or siloxanes present in the composition ranges from 30 to 70 % by weight in respect of the total amount of the composition, preferably ranges from 35 to 65 % by weight in respect of the total amount of the composition, more preferably ranges from 40 to 55 % by weight in respect of the total amount of the composition.

In a further embodiment of the composition of the second invention, the composition comprises at least a further emollient. Said further emollient is preferably a fatty acid ester, more preferably selected from cetyl palmitate, decyl oleate, ethyl oleate, isopropyl myristate, isopropyl palmitate, ethylhexyl stearate and mixtures thereof, even more preferably the composition comprises ethylhexyl stearate.

In a preferred embodiment of the composition of the second invention, the total amount of fatty acid ester or fatty acid esters present in the composition ranges from 1 to 10 % by weight in respect of the total amount of the composition, preferably ranges from 3 to 7 % by weight in respect of the total amount of the composition.

In a further embodiment of the composition of the second invention, said composition further comprises tea tree oil. In a preferred embodiment, the total amount of tea tree oil present in the composition ranges from 0.5 to 5% % by weight in respect of the total amount of the composition, preferably ranges from 1 to 3 % by weight in respect of the total amount of the composition.

In a further embodiment, the composition of the second invention as herein disclosed comprises at least one further ingredient which is selected from the group consisting of Ribes nigrum (blackcurrant) seed oil, Helianthus annuus seed oil, Helianthus annuus seed oil unsaponifiables, Cardiospermum halicacabum flower, leaf or vine extract, Rosmarinus officinalis (rosemary) plant, flower or leaf extract, Magnolia officinalis bark extract, Magnolia grandiflora bark extract, Vitis vinifera seed extract (e.g. caprylic/capric triglyceride), Echium plantagineum seed oil, Centella asiatica extract, Camelia sinensis leaf extract, Melia azadirachta leaf extract, Withania somnifera root extract, Aloe barbadensis extract (preferably leaf juice), corn (zea mays) oil, linoleic acid, linolenic acid, oleic acid, palmitic acid, stearic acid, eicosapentaenoic acid (i.e. EPA), docosahexaenoic acid (i.e. DHA), azelaic acid, myristic acid and any mixture thereof. Preferably, said composition comprises at least one further ingredient which is selected from the group consisting of Ribes nigrum (blackcurrant) seed oil, Helianthus annuus seed oil, Helianthus annuus seed oil unsaponifiables, Cardiospermum halicacabum flower, leaf or vine extract, Rosmarinus officinalis (rosemary) plant, flower or leaf extract, Magnolia officinalis bark extract, Vitis vinifera seed extract (e.g. caprylic/capric triglyceride), Echium plantagineum seed oil, corn (zea mays) oil, linoleic acid, linolenic acid and any mixture thereof.

Preferably, the total amount of said at least one further ingredient present in the composition ranges from 0.2 to 5% by weight in respect of the total amount of the composition, more preferably the total amount of said at least one further ingredient present in the composition ranges from 0.5 to 4% by weight in respect of the total amount of the composition.

Said at least one further ingredient more preferably comprises Ribes nigrum (blackcurrant) seed oil, Helianthus annuus seed oil and Rosmarinus officinalis (rosemary) plant, flower or leaf extract; more preferably, said at least one further ingredient comprises Ribes nigrum (blackcurrant) seed oil, Helianthus annuus seed oil, Rosmarinus officinalis (rosemary) plant, flower or leaf extract and Cardiospermum halicacabum flower, leaf or vine extract; even more preferably, said at least one further ingredient comprises Ribes nigrum (blackcurrant) seed oil, Helianthus annuus seed oil, Helianthus annuus seed oil unsaponifiables, Rosmarinus officinalis (rosemary) plant, flower or leaf extract and Cardiospermum halicacabum flower, leaf or vine extract.

In another preferred embodiment, said at least one further ingredient comprises Magnolia officinalis bark extract and Vitis vinifera seed extract (e.g. caprylic/capric triglyceride). Also in a preferred embodiment, said at least one further ingredient may comprise Helianthus annuus seed oil, Cardiospermum halicacabum flower, leaf or vine extract, and Echium plantagineum seed oil.

Alternatively, said at least one further ingredient may preferably comprise linoleic acid (i.e. omega-6 essential fatty acid) and linolenic acid (i.e. omega-3 essential fatty acid).

The presence of any of these herbal or botanical extracts and/or essential fatty acids in this preferred embodiment may advantageously contribute to improve skin situation, particularly the scalp condition, especially in subjects with skin hypersensitivity, dry skin or atopic skin, who tend to suffer from itching, a burning feeling, reddening of the skin or even damaged skin surface, thus supporting a therapeutic treatment such as the killing of ectoparasites and/or their ova on a mammal subject, without these herbal or botanical extracts and/or essential fatty acids having a therapeutic action. Thus, the composition according to this preferred embodiment supports skin normalization simultaneously to the therapeutic effects provided by the active substances, namely the eradication of ectoparasites, especially lice.

In a further embodiment of the composition of the second invention, at least 75% of ectoparasites present are killed within 4 hours after a 10-minute exposure to the composition, preferably at least 75% of ectoparasites present are killed within 2 hours after a 10-minute exposure to the composition, preferably at least 75% of ectoparasites present are killed within 1 hour after a 10-minute exposure to the composition, more preferably at least 75% of ectoparasites present are killed within 30 minutes after a 10-minute exposure to the composition, even more preferably at least 75% of ectoparasites present are killed within 5 minutes after a 5-minute exposure to the composition.

In a preferred embodiment of the composition of the second invention, at least 80% of ectoparasites present are killed within 5 min after a 5-minute exposure to the composition, preferably at least 90% of ectoparasites present are killed within 5 minutes after a 5-minute exposure to the composition, more preferably at least 100% of ectoparasites present are killed within 5 minutes after a 5-minute exposure to the composition.

In a more preferred embodiment of the composition of the second invention, at least 80% of ectoparasites present are killed within 5 min after a 2-minute exposure to the composition, preferably at least 90% of ectoparasites present are killed within 5 minutes after a 2-minute exposure to the composition, more preferably at least 100% of ectoparasites present are killed within 5 minutes after a 2-minute exposure to the composition.

In a preferred embodiment of the composition of the second invention, the lice are adult lice, especially adult head lice.

The compositions of the second invention are also highly effective in killing the lice ova. In a further embodiment of the composition as herein disclosed, at least 60% of the ova are killed within 14 days after 10-minute exposure, preferably at least 80% of the ova are killed within 14 days after 10-minute exposure, more preferably at least 80% of the ova are killed within 14 days after 5-minute exposure, even more preferably at least 40% of the ova are killed within 14 days after 2-minute exposure.

It is provided a method of killing non-flying lice and/or their ova on a mammal subject comprising, topically administering to an area on the subject where ectoparasites or their ova are present a composition of the second aspect of the second invention.

In a further embodiment of the method of the third aspect of the second invention, the composition is applied for 30 minutes or less, preferably the composition is applied for 20 minutes or less, more preferably the composition is applied for 10 minutes or less, even more preferably the composition is applied for 5 min or less. In a preferred embodiment, the composition is applied for 2 minutes.

In a preferred embodiment, the mammal is preferably a human. In a more preferred embodiment, the lice are head lice, especially human head lice.

In a fourth aspect of the second invention, it is provided the use of an aliphatic hydrocarbon compound having a density of 0.7 to 0.9 g/cm$^3$ at 20 °C for the manufacture of a medicament for killing ectoparasites and/or their ova on a subject.

It is provided a kit for use in treating lice infestations comprising, the composition of the second aspect of the second invention in a package or other enclosure; and instructions describing how to use the items included in the kit to kill lice.

The second invention also provides kits for treating lice infestations. In various embodiments, the kits include a composition of the present invention in a package or other enclosure. In a further embodiment of the kit of the sixth aspect of the second invention, the kit further comprises a nit comb to assist in removing lice and their eggs from hair. The package can be a box, or may simply be a wrapping (preferably of a plastic) that surrounds the kit. The comb is preferably provided inside the package, but can also be attached to the outside of the package. In other embodiments, the kit includes shower cap. In preferred embodiments, the kit also contains instructions that describe how to use the items included in the kit to kill ectoparasites.

## EXAMPLES

The following examples illustrate various embodiment of the invention and are not intended to limit the invention in any way.

### Example 1 - Formulations with squalane

Squalane, cyclomethicone and ethylhexyl stearate were blended in a recipient at room temperature (22°C ± 2°C) with a continuous mixing during about 20 minutes at low to medium speed to prevent incorporation of air. The product can be stored in an enclosed cover stainless steel storage tank at controlled room temperatures of 15-30°C until packaging.

| Ingredients | Compositions (w/w %) | | | | | |
|---|---|---|---|---|---|---|
| | Ex 1.1 | Ex 1.2 | Ex 1.3 | Ex 1.4 | Ex 1.5 | Ex 1.6 |
| Squalane | 50 | 100 | 30 | 40 | 20 | 75 |
| Decamethylcyclopentasiloxane | 45 | | 65 | 55 | 75 | 20 |
| Ethylhexyl stearate | 5 | | 5 | 5 | 5 | 5 |

Stability studies have been performed and the stability of compositions above is confirmed up to 36 months.

### Example 2 - Formulations with other hydrocarbons

The following compositions are prepared in a similar manner to the ones in example 1. The compounds are blended in a recipient at room temperature (22°C ± 2°C) with a continuous mixing during about 20 minutes at low to medium speed to prevent incorporation of air. The product can be stored in an enclosed cover stainless steel storage tank at controlled room temperatures of 15-30°C until packaging.

| Ingredients | Compositions (w/w %) | | | | |
|---|---|---|---|---|---|
| | Ex 2.1 | Ex 2.2 | Ex 2.3 | Ex 2.4 | Ex 2.5 |
| Paraffin | 50 | | | | |
| Petroleum jelly | | 50 | | | |
| Isohexadecane | | | 50 | | |
| Isoeicosane | | | | 50 | |
| Squalene | | | | | 50 |
| Decamethylcyclopentasiloxane | 45 | 45 | 45 | 45 | 45 |
| Ethylhexyl stearate | 5 | 5 | 5 | 5 | 5 |

### Example 3 - Formulations with Tea Tree (Melaleuca alternifolia) oil

The following compositions were prepared in a similar manner to the ones in example 1. The compounds are blended in a recipient at room temperature (22°C ± 2°C) with a continuous mixing during about 20 minutes at low to medium speed to prevent incorporation of air. The product can be stored in an enclosed cover stainless steel storage tank at controlled room temperatures 15-30°C until packaging.

| Ingredients | Compositions (w/w %) | | |
|---|---|---|---|
| | Ex 3.1 | Ex 3.2 | Ex 3.3 |
| Squalane | 99 | 50 | |
| Isopropyl myristate | | | 50 |
| Cyclomethicone | | 45 | 45 |
| Ethylhexyl stearate | | 4 | 4 |
| Tea Tree oil | 1 | 1 | 1 |

**Example 4 - Comparative examples with some pediculicide compositions in the market.**

| Ingredients | Compositions (w/w %) | | |
|---|---|---|---|
| | Ex 4.1 | Ex 4.2 | Ex 4.3 |
| Isopropyl myristate | 50 | | |
| Cyclomethicone | 50 | | |
| Permethrin | | 1.5 | |
| Decamethylcyclopentasiloxane | | | 5.0 |
| Octamethyltrisiloxane | | | 4.0 |
| Other non-active excipients | | 98.5 | 91 |

**Example 5** - **Formulations with at least one further ingredient especially suitable for subjects with skin hypersensitivity, dry skin or atopic skin**

The following compositions were prepared in a similar manner to the ones in example 1. The compounds are blended in a recipient at room temperature (22°C $\pm$ 2°C) with a continuous mixing during about 20 minutes at low to medium speed to prevent incorporation of air. The product can be stored in an enclosed cover stainless steel storage tank at controlled room temperatures 15-30°C until packaging.

| Ingredients | Compositions (w/w %) | | | | | | | |
|---|---|---|---|---|---|---|---|---|
| | Ex. 5.1 | Ex. 5.2 | Ex. 5.3 | Ex. 5.4 | Ex. 5.5 | Ex. 5.6 | Ex. 5.7 | Ex. 5.8 |
| Squalane | 30.000 | 30.000 | 30.000 | 30.000 | 30.000 | 30.000 | 30.000 | 30.000 |
| Decamethylcyclopentasiloxane | 61.000 | 64.000 | 63.000 | 63.000 | 63.000 | 61.000 | 62.000 | 60.000 |
| Ethylhexyl stearate | 5.000 | 5.000 | 5.000 | 5.000 | 5.000 | 5.000 | 5.000 | 5.000 |
| Ribes nigrum Seed Oil | 0.720 | - | 0.360 | - | - | - | - | - |
| Helianthus annuus Seed Oil Unsaponifiables | 0.100 | - | 0.050 | - | - | - | - | - |
| Cardiospermum halicacabum flower/leaf/vine extract | 0.020 | - | 0.010 | 0.010 | - | - | 0.010 | 0.010 |
| Tocopherol | 0.020 | - | 0.010 | 0.002 | - | - | 0.002 | 0.005 |
| Helianthus annuus seed oil | 0.020 | - | 0.010 | 0.060 | - | - | 0.060 | 0.060 |
| Rosmarinus officinalis leaf extract | 0.004 | - | 0.002 | - | - | - | - | - |
| Magnolia officinalis bark extract | - | 0.005 | - | - | - | - | 0.005 | - |
| Caprylic/capric triglyceride (Vitis vinifera seed extract) | - | 0.995 | - | - | - | - | 0.995 | - |
| Echium plantagineum seed oil | - | - | - | 0.160 | - | - | 0.160 | 0.160 |
| Zea mays oil (corn oil) | - | - | - | - | 2,000 | 4,000 | - | - |
| Linoleic acid | - | - | - | - | - | - | - | 2.982 |
| Linolenic acid | - | - | - | - | - | - | - | 0.015 |
| Octyldodecanol | 3.116 | - | 1.558 | 1.768 | - | - | 1.768 | 1.768 |

**Example 6 - Siloxane-free formulations**

The following compositions were prepared in a similar manner to the ones in example 1. The compounds are blended in a recipient at room temperature (22°C $\pm$ 2°C) with a continuous mixing during about 20 minutes at low to medium speed to prevent incorporation of air. The product can be stored in an enclosed cover stainless steel storage tank at controlled room temperatures 15-30°C until packaging.

| Ingredients | Compositions (w/w %) | | | | |
|---|---|---|---|---|---|
| | Ex. 6.1 | Ex. 6.2 | Ex. 6.3 | Ex. 6.4 | Ex. 6.5 |
| Squalane | 30.000 | 30.000 | 30.000 | 30.000 | 30.000 |
| Ethylhexyl stearate | 5.000 | 5.000 | 5.000 | 5.000 | 5.000 |
| Isohexadecane | 35.750 | 17.875 | - | 17.875 | - |
| Isododecane | 22.750 | 11.375 | - | 11.375 | - |
| C13-15 alkane | 6.500 | 35.750 | - | 3.250 | - |
| Diethylhexyl carbonate | - | - | 65.000 | 32.500 | 32.500 |
| Hydrogenated ethylhexyl olivate | - | - | - | - | 26.000 |
| Hydrogenated olive oil unsaponifiables | - | - | - | - | 6.500 |

**Example 7 - Procedure for evaluating ectoparasiticidal activity - efficacy against head lice crawling stages (late nymphs and adults)**

Biological test species: *Pediculus humanus capitis* De Geer, 1778 (head lice).

Stage and number: groups of 10 specimens; adults and nymphs of 3rd stage (males and females).

Head lice were collected from the heads of volunteers. Crawling stages were obtained by mechanical action of a nit comb and immediately settled in Petri dishes and incubated at 25°C and 60% RH until the time of testing, between three and five hours after collection.

Product samples were conditioned at the same environmental conditions as treatment application, at $25\pm1$ °C and $60\pm10$ % RH. Once treatments were applied, lice were incubated in the dark at the same environmental conditions. Treatments were conducted in 10 mL of product poured in 5 cm diameter Petri dishes. For negative controls, tap water was used instead and tests were prepared in the same way.

Crawling stages were transferred with great care and soft tweezers to the center of a Petri dish lined with moistened Whatman n° 1 filter paper with 0.1 mL of tap water. Only those specimens that moved from the center to the edge of the dish were used, being discarded those that remained motionless.

Lice treatment was conducted by complete immersion method for 5 minutes or 2 minutes by submerging each group of specimens into the product. Then, lice were transferred to a permeable well, washed with tap water for one minute with a wash bottle and settled in 5 cm Petri dishes previously labelled and lined with moistened filter paper Whatman n° 1 with 0.1 mL of tap water, before being introduced in the incubator until evaluation.

Three replicates were conducted for the treatments and three replicates with tap water were used as negative controls.

Crawling stages were observed under the binocular microscope at 5, 15, 30, 60, 180 minutes and 18 and 24 hours post-treatment, with a stereoscopic microscope Olympus SZ61TR with zoom up to 4.5x20 magnification. Mortality criteria with a unique observer were:

i) Alive: Head lice can walk, grab a hair and move forward.

ii) Vital signs apparent (VSA): Head lice seem to be apparently well but their movements are not coordinated; they can't walk nor grab a hair and move forward (moribund or knocked down).

iii) Vital signs reduced (VSR): Head lice show only slight movements on antennae, legs or digestive tract (moribund or knocked down).

iv) Dead (D): Complete movement cessation (including digestive tract).

Mortality percentage: For data analysis, the number of dead head lice is the result of the combination of knocked down (VSA and VSR) and dead (D). The percentage mortality is calculated in each replicate considering the number of alive specimens and affected specimens (VSA+VSR+D) following the relationship: *N° of affected specimens/ Total n° of*

*specimens* x *100*

**Example 8 - Effect of compositions on ectoparasites: efficacy against head lice crawling stages (late nymphs and adults)**

Example 8.1 (t=5 min immersion)

The results are summarized in Table 1.

Table 1: Mean mortality percentage and standard deviation (SD) of head lice crawling stages for composition of Example 1.1, in the control and treatment, at each recording points with 5 minutes immersion.

| Time post-application | % Mortality (Mean ± SD) | |
|---|---|---|
| | Control | Ex 1.1 |
| 5 min | 0 ± 0 | 100 ± 0 |
| 15 min | 0 ± 0 | 100 ± 0 |
| 30 min | 0 ± 0 | 100 ± 0 |
| 60 min | 0 ± 0 | 100 ± 0 |
| 180 min | 0 ± 0 | 100 ± 0 |
| 18 hours | 30 ± 10 | 100 ± 0 |
| 24 hours | 57 ± 12 | 100 ± 0 |

Table 2: Mean mortality percentage and standard deviation (SD) of head lice crawling stages of Examples 1.2, 3.1, 3.2 and 3.3, in the control and treatment, at each recording points with 5 minutes immersion.

| Time post-application | % Mortality (Mean ± SD) | | | | | |
|---|---|---|---|---|---|---|
| | Control[(1)] | Ex 1.2 | Ex 3.1 | Ex 3.2 | Control[(2)] | Ex 3.3 |
| 5 min | 0 ± 0 | 100 ± 0 | 100 ± 0 | 100 ± 0 | | |
| 15 min | 0 ± 0 | 100 ± 0 | 100 ± 0 | 100 ± 0 | 0 ± 0 | 100 ± 0 |
| 30 min | 0 ± 0 | 100 ± 0 | 97 ± 6 | 100 ± 0 | 0 ± 0 | 100 ± 0 |
| 60 min | 0 ± 0 | 100 ± 0 | 97 ± 6 | 100 ± 0 | 0 ± 0 | 100 ± 0 |
| 180 min | 0 ± 0 | 100 ± 0 | 97 ± 6 | 100 ± 0 | 0 ± 0 | 100 ± 0 |
| 18 hours | 20 ± 10 | 100 ± 0 | 100 ± 0 | 100 ± 0 | 23 ± 6 | 100 ± 0 |
| 24 hours | 50 ± 10 | 100 ± 0 | 100 ± 0 | 100 ± 0 | | |
| (1) Control results corresponding to Ex 1.2, 3.1 and 3.2. (2) Control results corresponding to Ex 3.3. | | | | | | |

Crawling stages efficacy in all the trials is 100% after five minutes of immersion at five minutes post-treatment application. Due to 100% mortality in the treatments at 18 and 24 hours, Abbot's correction does not apply.

Example 8.2 (t=2 min immersion)

The results obtained after 2-minute immersion are summarized in Table 3.

Table 3: Mean mortality percentage and standard deviation (SD) of head lice crawling stages, in the control and treatment, at each recording points with 2-minute immersion.

| Time post-application | % Mortality (Mean ± SD) | | | |
|---|---|---|---|---|
| | Control[(1)] | Ex 1.1 | Control[(2)] | Ex 1.3 |
| 5min | 0 ± 0 | 100 ± 0 | 0 ± 0 | 100 ± 0 |

(continued)

| Time post-application | % Mortality (Mean ± SD) | | | |
|---|---|---|---|---|
| | Control[1] | Ex 1.1 | Control[2] | Ex 1.3 |
| 15 min | 0 ± 0 | 100 ± 0 | 0 ± 0 | 100 ± 0 |
| 30 min | 0 ± 0 | 100 ± 0 | 0 ± 0 | 100 ± 0 |
| 60 min | 0 ± 0 | 100 ± 0 | 0 ± 0 | 100 ± 0 |
| 180 min | 0 ± 0 | 100 ± 0 | 0 ± 0 | 100 ± 0 |
| 18 hours | 45 ± 10 | 100 ± 0 | 20 ± 10 | 100 ± 0 |
| (1) Control results corresponding to Ex 1.1. (2) Control results corresponding to Ex. 1.3. | | | | |

Crawling stages efficacy is 100% after 2 minutes of immersion at five minutes post-treatment application. Due to 100% mortality at 18 hours in the treatments, Abbot's correction does not apply.

Example 8.3 - Comparatives formulas (t=5 min immersion)

Results observed after at different time post-application and obtained after 5-minute immersion of the formulas in the market are summarized in Table 4.

Table 4: Mean mortality percentage and standard deviation (SD) of head lice crawling stages for comparatives compositions of Example 4.2 and Example 4.3, in the control and treatment, at each recording points with 5 minutes immersion.

| Time post-application | % Mortality (Mean ± SD) | | |
|---|---|---|---|
| | Control | Ex 4.2 | Ex 4.3 |
| 60 min | 0 ± 0 | 17 ± 21 | 10 ± 10 |
| 180 min | 0 ± 0 | 13 ± 15 | 7 ± 12 |
| 6 hours | 0 ± 0 | 20 ± 10 | 17 ± 21 |
| 8 hours | 0 ± 0 | 20 ± 10 | 17 ± 21 |
| 18 hours | 0 ± 0 | 27 ± 6 | 27 ± 23 |
| 24 hours | 30 ± 10 | 47 ± 6 | 60 ± 20 |

The comparative compositions of examples 4.2 and 4.3 shown a mean pediculicide mortality of 27% at 18 hours post-treatment application. Even after 24 hours post-treatment application the mortality is of 47% and 60%, respectively. The efficacy of the formulas in the market is clearly much lower than the ones of the present invention.

**Example 9 - Procedure for Evaluating Compositions for ectoparasiticidal activity - efficacy against head lice ova**

Biological test species: *Pediculus humanus capitis* De Geer, 1778 (head lice).
Stage and number: groups of 10 ova.
Head lice ova were collected from volunteers, with Ethic Committee approval, by specialized staff by cutting the hair with round scissors, considering only those eggs viable (grey in colour) and located less than 1 cm from the scalp. Once obtained, the hair shafts with the eggs were settled in Petri dishes and incubated at 25°C and 60±10% relative humidity until the beginning of the assay, between twelve and fifteen hours after collection.
Product samples were conditioned at the same environmental conditions as treatment application, at 25±1°C and 60±10% RH. Once treatments were applied, the eggs were incubated in the dark at 29±1°C y 70±5% HR.
Treatments were conducted in 10 mL of product poured in 5 cm diameter Petri dishes. Negative controls, tap water, were prepared in the same way.
Ova were inspected, under a stereoscopic microscope Olympus SZ61TR with zoom up to 4.5x20 magnification, to determine the viability and development stage. Only those that showed to be in good shape, with an embryo and the surface and operculum intact, were selected. Then, they were classified in different development stages on behalf of their morphological characters as early (no differentiation) or late (red or black eye spot and appendages and/or embryonic movements). Once the number of viable ova available in each of the development stages was determined, these were distributed evenly in groups among replicates.

Ova treatment was conducted by complete immersion method for 5 minutes by submerging each group of hair shafts (3 cm) into the product. Then, the hair shafts were transferred to a permeable well and washed with tap water for one minute with a wash bottle and settled in 5 cm Petri dishes previously labelled and lined with moistened filter paper Whatman n° 1 with 0.1 mL of tap water, before being introduced in the incubator until evaluation.

Three replicates were conducted for the treatments and three replicates with tap water were used as negative controls.

Observations on the development of head lice eggs were made at 7 and 14 days after treatment, under a stereoscopic microscope Olympus SZ61TR with zoom up to 4.5x20 magnification.

Mortality criteria

Criteria used for data recording were:

i) Complete Hatching (CH): Vital lice hatch.
ii) Incomplete Hatching (IH): The operculum is opened but the louse is entirely or partially inside the egg.
iii) No hatching: With operculum closed, the egg is intact or shrunk. Development stage is stated as early stage (ES) or late stage (LS).

Mortality percentage

For data analysis, the number of dead ova is the result of the combination of incomplete hatching (IH) and not hatched, either in ES or LS. The percentage of mortality is calculated in each replicate considering the number of hatched ova and dead ova (ii,iii) following the relationship:

$$Number\ of\ dead\ ova\ /Total\ number\ of\ ova\ x\ 100.$$

The final value is obtained after correction with mortality in the controls using Abbot's formula:

$$Abbot\ correction = \frac{Mortality\ \%\ treatment - Mean\ \%\ mortality\ controls}{100 - Mean\ \%\ mortality\ controls}\ x\ 100$$

**Example 10 - Effect of compositions on ectoparasites: Efficacy against head lice ova**

Example 10.1 - t=5 minutes immersion

The results are summarized in Table 5.

Table 5: Ova mortality percentages obtained in each replicate of the treatments with the composition of Example 1.1, along with the mean percentage and standard deviations (SD) and the final percentage considering Abbot's correction.

| Replicate | % Mortality | | % Mortality after Abbott's correction | % Mortality | |
|---|---|---|---|---|---|
| | Control | Example 1.1 | | Control | Example 1.3 |
| R-1 | 20 | 100 | 100 | 0 | 40 |
| R-2 | 0 | 70 | 67.86 | 0 | 60 |
| R-3 | 0 | 90 | 89.29 | 0 | 50 |
| Mean ± SD | 6.67 ± 11.55 | 86.67 ± 15.28 | 85.71 ± 16.37 | 0 | 50.00 ± 10.00 |

The treatment with the compositions of Examples 1.1 and 1.3 show a mean ovicidal mortality of 87% and 50%, respectively, at 14 days post-treatment application. After applying Abbot's correction to the values obtained with Example 1.1, considering mortality in the controls, the final value is 86%. Due to zero mortality in controls with Example 1.3 at 14 days, Abbot's correction does not apply.

Example 10.2 - t=5 minutes immersion

In relation to the composition of Example 3.3, the results are summarized in Table 6.

Table 6: Ova mortality percentages obtained after 5 of immersion, along with the mean percentage and standard deviations (SD) and the final percentage considering Abbot's correction.

| | % Mortality (Mean ± SD) | | |
|---|---|---|---|
| Time of immersion | Control | Example 3.3 | % Mortality after Abbott's correction |
| 5 minutes | 6.7 ± 5.8 | 60.0 ± 10.0 | 53.4 ± 10.0 |

The treatment group shows a mean ovicidal mortality of 60% with 5 minutes immersion at 14 days post-treatment application. After applying Abbot's correction, considering mortality in the controls, the final value is 53.4%.

Example 10.3 - t=2 minutes immersion

The results are summarized in Table 7.

Table 7: Ova mortality percentages obtained in each replicate of the treatments with the composition of Example 1.1 and Example 1.3 after 2 min of immersion of the product.

| | % Mortality (Mean ± SD) | | | |
|---|---|---|---|---|
| Time of immersion | Example 1.1 | | Example 1.3 | |
| | | after Abbott's correction | | after Abbott's correction |
| 2 minutes | 43.33 ± 15.05 | 26.98 ± 24.96 | 56.67 ± 15.28 | 50.00 ± 17.63 |

The treatment with composition of Example 1.1 and 1.3 show a mean ovicidal mortality of 43% and 57%, respectively, at 14 days post-treatment application. After applying Abbot's correction, considering mortality in the controls, the final value is 27% and 50%, respectively.

Example 10.4 - Comparative formula

In relation to the compositions of comparative Example 4, the results are summarized in Table 8.

Table 8: Ova mortality percentages obtained after 5 minutes of immersion, along with the mean percentage and standard deviations (SD) and the final percentage considering Abbot's correction.

| | % Mortality (Mean ± SD) | | | | | | |
|---|---|---|---|---|---|---|---|
| Time of immersion | Control | Example 4.1 | | Example 4.2 | | Example 4.3 | |
| | | | after Abbott's correction | | after Abbott's correction | | after Abbott's correction |
| 5 minutes | 6.7 ± 5.8 | 13.3 ± 5.8 | 7.07 | 6.7 ± 11.5 | 0 | 3.3 ± 5.8 | 0 |

The composition of example 4.1 shows a mean ovicidal mortality of 13% at 14 days post-treatment application. After applying Abbot's correction, considering mortality in the controls, the final value is 7%. The other two formulas in the market after applying Abbot's correction did not show any ovicidal effect.
The efficacy of the formulas in the market is much lower than the ones of the present invention.

Example 10.5 - t=9 minutes immersion

In relation to the composition of Example 1.3, the results are summarized in Table 9.

Table 9: Ova mortality percentages obtained in each replicate of the treatments with the composition of Example 1.3, along with the mean percentage and standard deviations (SD) and the final percentage considering Abbot's correction.

| Replicate | % Mortality | |
|---|---|---|
| | Control | Example 1.3 |
| R-1 | 0 | 80 |
| R-2 | 0 | 90 |
| R-3 | 0 | 80 |
| Mean ± SD | 0 | 83.33 ± 5,77 |

The treatment group shows a mean ovicidal mortality of 83% with 9 minutes immersion at 14 days post-treatment application. Due to zero mortality in the controls at 14 days, Abbot's correction does not apply.

Example 10.6 - t=12 minutes immersion

In relation to the composition of Example 1.1, the results are summarized in Table 10.

Table 10: Ova mortality percentages obtained in each replicate of the treatments with the composition of Example 1.1, along with the mean percentage and standard deviations (SD) and the final percentage considering Abbot's correction.

| Replicate | % Mortality | |
|---|---|---|
| | Control | Example 1.1 |
| R-1 | 0 | 80 |
| R-2 | 0 | 100 |
| R-3 | 0 | 100 |
| Mean ± SD | 0 | 93.33 ± 11,55 |

The treatment group shows a mean ovicidal mortality of 93% with 12 minutes immersion at 14 days post-treatment application. Due to 0% mortality in the controls at 14 days, Abbot's correction does not apply.

**Example 11 - Clinical study on the sensitizing potential of the formulation in healthy adult volunteers according to Marzulli-Maibach method: human repeated insult patch test**

The main objective of this study was to verify irritation and sensitizing potential of the formulations of Example 1 after repeated applications under occlusion, 3 times a week during 3 consecutive weeks (Induction Phase), on the back of 50 healthy adult volunteers with different skin types (I to IV), followed by a 2-weeks rest period and a single application of the product under patch to a well delimited site and to the induction site (Challenge Phase).

The study showed no irritating potential during the Induction Phase of the study and no sensitizing potential during the Induction Phase.

Under the experimental study conditions, it is considered that the formulations of Example 1 are non-irritating and non-sensitizing.

**Example 12 - Clinical study to assess the skin tolerance under normal conditions of use in children from 1 year of age under dermatological and paediatric control: Use test**

The main objective of this study was to verify the absence of cumulative reactions and cumulative irritations (functional and physical signs) associated with the application of the formulations of Example 1 under normal conditions of use for 2 weeks in 20 healthy children of both sexes, aged between 1 and 3 years. This study was conducted under dermatological control (subjects individually examined by a Dermatologist Investigator) and paediatric (subjects individually examined by a Paediatric Investigator).

The experimental phase of this trial lasted for 14 days, in which the parents / guardians of the children made 3 visits: day 0, day 7 and day 14.

On days 0 and 7, the lotion was applied to the dry hair of the children by performing a gentle massage so that hair and scalp were well impregnated, insisting on the area behind the ears and the nape. The following amounts of the product were used depending on the type of hair:

- Short or very fine hair: 1/4 bottle
- Half mane: 1/2 boat
- Long or very thick hair: 1 bottle (100 ml)

After applying the product, the product was left for 5 minutes and then the hair was washed with the usual shampoo, rinsing with plenty of water. The hair was dried hair in the usual way.

In the 3 visits, the researchers evaluated the area of application of the product after its use to assess possible adverse reactions or irritation produced by the products. The products did not produce any undesirable skin reactions in the study participants after 14 days of use, according to the study's researchers. During the 14-day study period, none of the parents / guardians reported any undesirable reactions.

Under the experimental study conditions, it is considered that the formulations of Example 1 are very well tolerated at skin level and it can be stated that the product has been "tested under dermatological and paediatric control".

**Reference Example 13 - Procedure for evaluating ectoparasiticidal activity - efficacy against other lice species, mites, fleas and ticks.**

The conditions described in Example 9 are used for carrying out additional tests in order to assess the ectoparasiticidal activity of the compositions of the present invention on other ectoparasite species belonging to the same taxonomical order (i.e. suborder Anoplura) as *Pediculus humanus capitis* (head lice), namely, *Pediculus humanus corporis* (body lice) and *Phthirus pubis* (crab lice) in human subjects, and also *Haematopinus suis* (lice) in animal subjects, more specifically, in swine, since similar results were to be expected for species of the same taxonomical order in terms of excellent ectoparasiticidal action with the compositions of the present invention.

In this regard, genetic tools have made it possible to first separate human lice into head and body lice, and then determine that the main differences between head and body lice mainly concern gene expression levels. Thus, while head and body lice almost have the same genomic content, they are phenotypically different as a result of differential gene expression, and it has been possible to determine the existence of 14 putative differentially expressed genes between head and body lice.

Also, a study based on the 18S rRNA gene, as well as subsequent studies focused on mitochondrial genes and intergenic spacers, have revealed that there are three clades of head lice, one of which may also be body lice (clade A), Based on all these studies, it is suggested that clade A head louse originated from the body louse, and has a deleted genome.

Additional tests are also conducted on *Sarcoptes scabiei* (mites) in human subjects, *Rhipicephalus microplus* (formerly known as *Boophilus microplus;* also known as cattle tick) in cattle, and *Pulex irritans* (fleas) in dogs.

**Example 14 - Clinical studies to assess mechanism of action of the compositions of the invention**

Two experiments were performed to assess the mechanism of action of the invention using a methodology based on scientific literature.

Example 14.1 - Experiment 1

Biological test species: *Pediculus humanus capitis* De Geer, 1778 (head lice).

Stage and number: groups of 5 specimens; adults and nymphs of 3rd stage (males and females); groups of 5 nits or eggs.

Procedure:

Tracer solutions: The penetration or absence of penetration of Ex. 1.1 (50% squalane, 45% decamethylcyclopentasiloxane, 5% ethylhexyl stearate), one of the compositions of the invention, through the spiracles in the respiratory system and / or cuticle of lice was studied using dyes dissolved in absolute ethanol (positive control, based on Candy, K., Brun, S ., Nicolas, P., Durand, R., Remi N. Charrel, RN, Izri, A. Parasite 2018, 25, 8), and dyes dissolved together with the composition of the invention.

Immersions: Each group of lice was introduced into 5 ml Eppendorf tubes containing 1 ml of coloured ethanol and 1 ml of the coloured composition of the invention for five different times: 2 minutes, 10 minutes, 30 minutes, 2, 4 and 12 hours. Regarding nits, the same methodology was used, but at three different times of immersion: 5 minutes, 2 and 24 hours. Once the immersions were done, lice and nits were dried on a filter paper to absorb the excess of fluid and washed vigorously with tap water before examining them under a binocular microscope.

Results:

The observed tracing or circulation in mobile stages (adult lice and nymphs) observed when using the composition of the invention is comparable and similar to that observed with ethanol (positive control) and the results obtained in Candy *et al.* 2018, that is, it penetrates through the spiracles flooding the entire respiratory system and also penetrates the entire body through the cuticle of the louse (head, abdomen, legs).

Regarding nits, the composition of the invention fixes externally covering the section of the operculum corresponding to the aeropils, through which the air that allows embryo respiration passes, and the hatching suture, which is torn to allow hatching.

Example 14.2 - Experiment 2

Biological test species: *Pediculus humanus capitis* De Geer, 1778 (head lice).

Stage and number: groups of 10 specimens; adults and nymphs (males and females).

Procedure:

Treatments were conducted in 10 mL of product poured in 5 cm diameter Petri dishes. For negative controls, tap water was used instead and tests were prepared in the same way.

Crawling stages were carefully transferred using soft tweezers to the center of a Petri dish lined with moistened Whatman n° 1 filter paper with 0.1 mL of tap water.

Lice treatment was conducted by complete immersion method by submerging each group of specimens into the composition of Example 1.1 of the invention for 2 minutes. Lice were then transferred to a permeable well, washed with tap water for one minute with a wash bottle and settled in 5 cm Petri dishes previously labelled and lined with moistened filter paper Whatman n° 1 with 0.1 mL of tap water, before being introduced in the incubator until evaluation.

3 replicates were conducted using the composition of Example 1.1 of the invention and 3 replicates with tap water were used as negative controls.

Crawling stages were observed under the binocular microscope at 5, 15, 30, 60, 180 minutes and 18 and 24 hours post-treatment, with a stereoscopic microscope Olympus SZ61TR with zoom up to 4.5x20 magnification.

Results:

Observation of lice immersion in the composition of the invention with the binocular microscope, and the measured time it takes for any apparent movement to stop (except for peristaltic movements of the intestine) indicate that after 60-90 seconds of immersion in the composition of the invention, lice are affected in such a way that signs of recovery are not subsequently observed, even after being washed.

In some specimens, what appears to be the rupture of the digestive tract can be observed 15 minutes after treatment, since there is a band of blood overflowing parallel to it. As time passes, that is, in the observations of the second interval, this overflow diffuses through the internal cavity, observing this effect in both adults and nymphs in a similar way. The blood that is released by the rupture of the intestinal tract can, sometimes, also be dispersed over the thorax and legs.

Conclusions

Taking into account the results of the experiments described above and the bibliography, and without wishing to be bound by theory, it is herein postulated that the mechanism of action of the invention in relation to the mortality in mobile stages of head louse is based on the provocation of osmotic stress linked to the retention or loss of water by blockage of respiratory structures (i.e. spiracles and trachea) and penetration through the cuticle of the exoskeleton, also causing the rupture of the digestive tract.

Regarding eggs or nits, the uniform coating of the aeropiles allows to hypothesize that the product acts by externally blocking the entry of air.

Therefore, it is suggested that the pediculicidal activity of the invention would be due to a physical mechanism of action on lice and nits.

**Claims**

1. Squalane for use in a therapeutic method of killing lice and/or their ova on a mammal subject.

2. A composition for use in a therapeutic method of killing lice and/or their ova on a mammal subject, wherein squalane is present in an amount of at least 5 % by weight in respect of the total amount of the composition.

3. The composition for use according to claim 2, wherein squalane is present in an amount of more than 20 % by weight in respect of the total amount of the composition.

4. The composition for use according to claim 3, further comprising at least a siloxane.

5. The composition for use according to claim 4, wherein the total amount of siloxane or siloxanes present in the composition ranges from 30 to 70 % by weight in respect of the total amount of the composition.

6. The composition for use according to any one of claims 2 to 5, wherein the composition comprises at least a further emollient.

7. The composition for use according to claim 6, wherein the further emollient is a fatty acid ester.

8. A topical composition comprising squalane and at least a siloxane, wherein squalane is present in an amount of at least 30% by weight in respect of the total amount of the composition.

9. The composition according to claim 8, wherein the total amount of siloxane or siloxanes present in the composition ranges from 30 to 70 % by weight in respect of the total amount of the composition.

10. The composition according to any claim 8 or 9, wherein the composition is for use in a therapeutic method of killing lice and/or their ova on a mammal subject.

11. A kit for use in treating lice infestations comprising squalane or a composition as defined in any one of claims 2 to 10 in a package or other enclosure; and instructions describing how to use the items included in the kit to kill lice.

12. A composition for use in a therapeutic method of killing lice and/or their ova on a mammal subject, the composition comprising an aliphatic hydrocarbon compound having a density of 0.7 to 0.9 g/cm$^3$ at 20 °C and at least a siloxane having a viscosity less than 90 cSt, wherein the hydrocarbon compound is present in an amount of at least 5% by weight in respect of the total amount of the composition, and wherein the aliphatic hydrocarbon compound is squalane.

13. The composition for use according to claim 12, wherein the total amount of siloxane or siloxanes present in the composition ranges from 30 to 70 % by weight in respect of the total amount of the composition.

**Patentansprüche**

1. Squalan zur Verwendung in einem therapeutischen Verfahren zum Abtöten von Läusen und/oder ihren Eizellen bei einem Säugetier.

2. Eine Zusammensetzung zur Verwendung in einem therapeutischen Verfahren zum Abtöten von Läusen und/oder ihren Eizellen bei einem Säugetier, wobei das Squalan in einer Menge von mindestens 5 Gew.- % in Bezug auf die Gesamtmenge der Zusammensetzung vorhanden ist.

3. Die Zusammensetzung zur Verwendung nach Anspruch 2, wobei das Squalan in einer Menge von mehr als 20 Gew.- %, in Bezug auf die Gesamtmenge der Zusammensetzung, vorhanden ist.

4. Die Zusammensetzung zur Verwendung nach Anspruch 3, ferner umfassend mindestens ein Siloxan.

5. Die Zusammensetzung zur Verwendung nach Anspruch 4, wobei die Gesamtmenge an Siloxan oder Siloxanen, die in der Zusammensetzung vorhanden sind, von 30 bis 70 Gew.- %, in Bezug auf die Gesamtmenge der Zusammensetzung, liegt.

6. Die Zusammensetzung zur Verwendung nach einem der Ansprüche 2 bis 5, wobei die Zusammensetzung mindestens ein weiteres Emolliens umfasst.

7. Die Zusammensetzung zur Verwendung nach Anspruch 6, wobei das weitere Emolliens ein Fettsäureester ist.

8. Eine topische Zusammensetzung umfassend Squalan und mindestens ein Siloxan, wobei das Squalan in einer Menge von mindestens 30 Gew.- % in Bezug auf die Gesamtmenge der Zusammensetzung vorhanden ist.

9. Die Zusammensetzung nach Anspruch 8, wobei die Gesamtmenge an Siloxan oder Siloxanen, die in der Zusammensetzung vorhanden sind, von 30 bis 70 Gew.- %, in Bezug auf die Gesamtmenge der Zusammensetzung, liegt.

10. Die Zusammensetzung nach einem der Ansprüche 8 oder 9, wobei die Zusammensetzung zur Verwendung in einem therapeutischen Verfahren zum Abtöten von Läusen und/oder ihren Eizellen an einem Säugetier bestimmt ist.

11. Ein Kit zur Verwendung bei der Behandlung von Läusebefall, umfassend Squalan oder eine Zusammensetzung wie in einem der Ansprüche 2 bis 10 definiert in einer Verpackung oder einer anderen Umhüllung; und Anweisungen, die beschreiben, wie die in dem Kit enthaltenen Gegenstände verwendet werden sollen, um Läuse abzutöten.

12. Eine Zusammensetzung zur Verwendung in einem therapeutischen Verfahren zum Abtöten von Läusen und/oder ihren Eizellen an einem Säugetier, wobei die Zusammensetzung eine aliphatische Kohlenwasserstoffverbindung mit einer Dichte von 0,7 bis 0,9 g/cm$^3$ bei 20 °C und mindestens ein Siloxan mit einer Viskosität von weniger als 90 cSt umfasst, wobei die Kohlenwasserstoffverbindung in einer Menge von mindestens 5 Gew.- % in Bezug auf die Gesamtmenge der Zusammensetzung vorhanden ist und wobei die aliphatische Kohlenwasserstoffverbindung Squalan ist.

13. Die Zusammensetzung zur Verwendung nach Anspruch 12, wobei die Gesamtmenge an Siloxan oder Siloxanen, die in der Zusammensetzung vorhanden sind, von 30 bis 70 Gew.- %, in Bezug auf die Gesamtmenge der Zusammensetzung, liegt.

**Revendications**

1. Squalane destiné à être utilisé dans un procédé thérapeutique pour tuer les poux et/ou leurs œufs sur un sujet mammifère.

2. Une composition destinée à être utilisée dans un procédé thérapeutique pour tuer les poux et/ou leurs œufs sur un sujet mammifère, dans laquelle le squalane est présent en une quantité d'au moins 5 % en poids par rapport à la quantité totale de la composition.

3. La composition destinée à être utilisée selon la revendication 2, dans laquelle le squalane est présent en une quantité supérieure à 20 % en poids par rapport à la quantité totale de la composition.

4. La composition destinée à être utilisée selon la revendication 3, comprenant en outre au moins un siloxane.

5. La composition destinée à être utilisée selon la revendication 4, dans laquelle la quantité totale de siloxane ou de siloxanes présent(s) dans la composition va de 30 à 70 % en poids par rapport à la quantité totale de la composition.

6. La composition destinée à être utilisé selon l'une quelconque des revendications 2 à 5, dans laquelle la composition comprend au moins un autre émollient.

7. La composition destinée à être utilisée selon la revendication 6, dans laquelle l'autre émollient est un ester d'acide gras.

8. Une composition topique comprenant du squalane et au moins un siloxane, dans laquelle le squalane est présent en une quantité d'au moins 30 % en poids par rapport à la quantité totale de la composition.

**9.** La composition selon la revendication 8, dans laquelle la quantité totale de siloxane ou de siloxanes présent(s) dans la composition va de 30 à 70 % en poids par rapport à la quantité totale de la composition.

**10.** La composition selon l'une quelconque des revendications 8 ou 9, dans laquelle la composition est destinée à être utilisée dans un procédé thérapeutique pour tuer les poux et/ou leurs œufs sur un sujet mammifère.

**11.** Un kit destiné à être utilisé dans le traitement des infestations par les poux comprenant du squalane ou une composition telle que définie dans l'une quelconque des revendications 2 à 10 dans un emballage ou une autre enveloppe ; et des instructions décrivant comment utiliser les articles inclus dans le kit pour tuer les poux.

**12.** Une composition destinée à être utilisée dans un procédé thérapeutique pour tuer les poux et/ou leurs œufs sur un sujet mammifère, la composition comprenant un composé hydrocarboné aliphatique ayant une densité de 0,7 à 0,9 g/cm$^3$ à 20 °C et au moins un siloxane ayant une viscosité inférieure à 90 cSt, dans laquelle le composé hydrocarboné est présent en une quantité d'au moins 5 % en poids par rapport à la quantité totale de la composition, et dans laquelle le composé hydrocarboné aliphatique est le squalane.

**13.** La composition destinée à être utilisée selon la revendication 12, dans laquelle la quantité totale de siloxane ou de siloxanes présent(s) dans la composition va de 30 à 70 % en poids par rapport à la quantité totale de la composition.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 17382439
- US 4147800 A
- US 6303581 B
- GB 2204243 B2
- WO 0001347 A
- WO 2001019190 A
- WO 2001040446 A
- WO 03092583 A
- US 20130018016 A1

### Non-patent literature cited in the description

- **DAVID C. FLINDERS et al.** Pediculosis and Scabies. *American Family Physician*, 2004, vol. 69, 342-344
- **J. ROSE**. The Aromatherapy Book - Applications & Inhalations. North Atlantic Books, 1992
- **CANDY, K.** ; **BRUN, S .** ; **NICOLAS, P.** ; **DURAND, R.** ; **REMI N.** ; **CHARREL, RN** ; **IZRI, A.** *Parasite*, 2018, vol. 25, 8